# EUROPEAN PATENT APPLICATION

(11) **EP 3 725 340 A1**
(43) Date of publication of application: **21.10.2020**
(21) Application number: 19170404.8
(22) Date of filing: 19.04.2019
(51) Int. Cl.: A61M 1/06

(54) **CUSHION CONFIGURED TO BE MOUNTED TO AN AIR PASSAGE ELEMENT OF A BREAST PUMP DEVICE**

(71) Applicant: Koninklijke Philips N.V., 5656 AG Eindhoven (NL)
(72) Inventor: GOSENSHUIS, Daan Hendrik, 5656 AE Eindhoven (NL); CLAASSEN, Coen Petrus Martinus, 5656 AE Eindhoven (NL); DOBRUSSKIN, Christoph, 5656 AE Eindhoven (NL)
(74) Representative: de Haan, Poul Erik

(57) **Abstract**

Complexity of a process of mounting a cushion (40) to an air passage element (20) of a breast pump device is decreased by only requiring a user to arrange the cushion (40) on the air passage element (20) in a first arrangement in which the cushion (40) is easily accessible. Particularly, the user does not need to perform cumbersome actions aimed at accurately putting the cushion (40) to an intended, correct position, as a predetermined mounting arrangement of the cushion (40) on the air passage element (20) is realized under the influence of underpressure prevailing at a back side of the air passage element (20). In the process, the cushion (40) is put to the predetermined mounting arrangement, wherein the cushion (40) is made to flex and/or move.

## Description

### FIELD OF THE INVENTION

In one aspect, the invention relates to a generally sleeve-shaped cushion that is configured to be mounted to an air passage element of a breast pump device and to contact a breast of a user of the breast pump device.

In another aspect, the invention relates to an assembly of a cushion as mentioned in the foregoing and a breast pump device comprising an air passage element.

In yet another aspect, the invention relates to breast pump device that comprises an expression unit including an air passage element, the expression unit being designed to allow a cushion that is generally sleeve-shaped and that comprises a breast-facing section at one side thereof and a pump section at another side thereof to be mounted to the air passage element.

### BACKGROUND OF THE INVENTION

In general, a breast pump device is a well-known tool for extracting milk from a breast of a lactating woman, or from two breasts simultaneously. Breast pump devices may be used in various situations, for example, if a baby or infant is physically not capable of extracting milk from the breast, or if a mother is separated from her baby or infant and the baby or infant is to be fed with breast milk at a later stage, by the mother or another person. Hence, breast pump devices are used by women to express breast milk at a convenient time, to be stored for later consumption by their/a baby or infant. Breast pump devices may also be helpful in a situation in which it is desired to stimulate and increase milk production in women with a low milk supply or to relieve pressure from engorged breasts.

A breast pump device typically includes one or two expression units. The breast pump device may be of an integral design, in which case the device may be suitable to be worn on the body, or the breast pump device may comprise one or two separate kits commonly known as expression kits. Among other things, an expression unit comprises an air passage element which may include a breast-receiving funnel for receiving at least a part of a woman's breast, which air passage element is designed for connection to a vacuum unit for realizing a pressure cycle in the expression unit, by means of which milk expression from the breast is enabled. In practical cases, the vacuum unit comprises an electric vacuum pump device, but manually operated breast pump devices are also known and used in practice. The fact is that by generating a pressure cycle, particularly a vacuum cycle, possibly accompanied by a certain way of massaging the breast, a simulation of a feeding action is obtained, which triggers the necessary let-down reflex in the lactating woman using the breast pump device. For the sake of completeness, it is noted that the term "vacuum" as used in this text refers to a relatively low pressure, i.e. a pressure that is significantly lower than ambient pressure.

It is known to apply a cushion between the breast/areola/nipple tissue and the air passage element, and to thereby increase comfort and effectiveness of the use of a breast pump device. Advantageously, such a cushion comprises a material that is known as being soft and flexible. In this respect, silicone is a practical example of a useful material.

WO 2014/135504 A1 relates to a breast interface for use in a breast pump device. The breast interface includes a flexible liner and a generally rigid liner support frame. The liner and the liner support frame may typically be formed as two separate structural components that need to be assembled together upon use to form the breast interface, and that may be taken apart afterwards to facilitate cleaning and replacement of one of the components if so desired. The liner and the liner support frame may alternatively be integrally formed as a single structural component.

In case the breast interface comprises the liner and the liner support frame as separate structural components, the breast interface may be assembled as follows. A user may axially insert a tubular rear section of the liner through a front ring of the liner support frame and reach through one or more access windows provided in the liner support frame in order to grip a flange or back ring provided on the rear section of the liner. Holding the flange or back ring, the user may push or pull the liner rearward in the liner support frame until the rear section of the liner snaps in place at a defined axial position. To complete the assembly, the user may verify that the front ring of the liner support frame has been properly received in an annular groove in a front section of the liner, and, where necessary, still ensure proper seating of the front ring therein, preferably such that the front ring is flush with a rear end of at least one the wall parts that define the groove.

In general, cushions for use with breast pump devices need to be installed at a well-defined position yet are difficult to assemble intuitively towards such position due to their flexibility. It follows from the above explanation of what is known from WO 2014/135504 A1 that installing a cushion may require quite a number of complex actions. Complex installation procedures are not only cumbersome to a user, but also involve an increased chance that a cushion does not end up at the intended position, as a result of which leakage of pressure may occur when the breast pump device is operated so that the milk expression process may be rendered ineffective. Further, a need to use her fingers for putting a cushion to the proper position is perceived by a user as unhygienic, especially when touching an interior surface of the cushion cannot be avoided in the process.

In view of the foregoing, it is an object of the invention to provide a cushion of a new design, particularly a new design which allows for easy mounting of the cushion to an air passage element of a breast pump device in a correct manner.

### SUMMARY OF THE INVENTION

The invention provides a cushion that is configured to be mounted to an air passage element of a breast pump device and to contact a breast of a user of the breast pump device. The cushion is generally sleeve-shaped and comprises a breast-facing section at one side thereof and a pump section at another side thereof, wherein the pump section includes a contacting surface that is configured to contact a surface of the air passage element. Further, the cushion is configured to be deformable from an initial configuration to a final configuration when the cushion is brought under the influence of underpressure acting on the cushion from the side of the pump section.

The invention involves the insight that by providing a cushion that is deformable under the influence of a pressure difference, it is possible to create a situation in which it is sufficient for a user to perform an action of accurately positioning the cushion at a front side of the air passage element only, as the cushion may be automatically put to an intended mounting arrangement in the air passage element under the influence of underpressure. For example, the cushion may be pulled deeper into the air passage element in the process. Hence, the invention makes clever use of the fact that a breast pump device is adapted to generate underpressure at a back side of the air passage element. Also, when the invention is applied, a user is allowed to manipulate the cushion while the cushion is in a relatively compact condition, without any need for the user to touch the cushion's interior.

In the context of the invention, i) the initial configuration of the cushion may be a compressed configuration and the final configuration of the cushion may be an uncompressed configuration, ii) the initial configuration of the cushion may be an uncompressed configuration and the final configuration of the cushion may be a stretched configuration, or iii) the initial configuration of the cushion may be a compressed configuration and the final configuration of the cushion may be a stretched configuration, to mention a number of examples of practical possibilities. In any case, the cushion of the invention allows for a procedure of mounting the cushion to an air passage element of a breast pump device according to which a user handles the cushion in the initial, relatively compact configuration, and puts the cushion at an appropriate position with respect to the air passage element without needing to touch the cushion's interior and/or to put one or more fingers at places which are hard to reach. The remainder of the mounting procedure is performed automatically as soon as underpressure is created in the breast pump device, either especially for the purpose of ensuring that the cushion is deformed for the purpose of reaching the final configuration and is thereby automatically installed on the air passage element in the correct way, or for the purpose of realizing the pressure cycle that is needed for invoking milk expression, in which case the automatic and correct installation of the cushion following from deforming the cushion is achieved as a most advantageous side effect.

The invention includes an option of providing a suitable type of protective cover to be positioned with respect to the air passage element for delimiting a vacuum space during the automatic cushion installation procedure and thereby enhancing effectiveness of the procedure. Also, the invention includes an option according to which the cushion comprises a valve located in the cushion to at least hinder an airflow through the cushion, which can be applied to decrease or even totally avoid a loss of pressure through the cushion during a procedure of putting the cushion to an intended mounting arrangement under the influence of an underpressure acting on the cushion from the side of the pump section.

The cushion according to the invention may particularly be stretchable in a generally axial direction and/or any other appropriate direction. The cushion may comprise a suitable flexible material such as silicone, thermoplastic elastomer, or natural rubber in at least an appropriate part thereof. Preferably, along a portion of its length, the cushion is flexible in the radial direction as well, to such an extent that the cushion is allowed to locally collapse and to thereby perform a stimulating action on a breast that may be beneficial to the effectiveness of milk expression from the breast. The cushion can have any suitable shape. The fact that the cushion is denoted as being generally sleeve-shaped is to be understood so as to imply that the cushion has an internal passage for allowing air to flow through and has something like a sleeve wall at least partially encompassing the internal passage. The sleeve wall can be of any appropriate design, and any practical distribution of values of both an inner diameter and an outer diameter of the sleeve wall along a length of the sleeve wall may be applicable.

The cushion according to the invention can be of a type designed to allow inside mounting on an air passage element of a breast pump device, a type designed to allow outside mounting on such an air passage element, or a type designed to allow both inside mounting and outside mounting on such an air passage element. Hence, the cushion may be configured to be at least partially arranged in an air passage element of a breast pump device and/or to be arranged to at least partially encompass such an air passage element, wherein the contacting surface may be configured to contact an interior surface of the air passage element and/or to contact an exterior surface of the air passage element.

It is beneficial if the shape of the cushion is adapted to the general shape of (a part of) a breast and/or the general shape of an actual air passage element. For example, the cushion may of a design in which at least a part of the breast facing section tapers in a direction towards the pump section.

In the case of the cushion being stretchable in a generally axial direction, the cushion's ability to stretch under the influence of underpressure exerted from the side of the pump section may be realized in any practical way. For example, the cushion may be equipped with a stretching section at a position between the breast-facing section and the pump section, the stretching section being deformable between a more compact configuration and a more straightened configuration and thereby being configured to facilitate stretching of the cushion. The stretching section may include one or more folded areas of which the folds can be smoothed to a certain extent.

In the context of the invention, it is advantageous if measures are taken in order to prevent a collapse of contact between the contacting surface of the cushion and a surface of an air passage element once the cushion has been positioned with respect to the air passage element. In this respect, it is noted that it may be beneficial for the pump section to include a stiffened annular portion and for the contacting surface to be constituted by at least a portion of a surface of the pump section at a position of the stiffened annular portion. Such a stiffened annular portion of the pump section may be realized as a portion of the pump section where the pump section is locally thickened.

It may be practical if the breast-facing section includes a coupling portion that is configured to engage with the air passage element, as having a possibility of coupling the cushion to the air passage element may help in correctly positioning the cushion with respect to the air passage element and/or avoiding unintended displacement of the cushion under the influence of contact with a breast, etc. In a practical embodiment, the coupling portion comprises a groove configured to receive and accommodate a peripheral front edge of the air passage element. Further, the coupling portion may comprise a catch of a suitable design, for example.

The invention also relates to an assembly of a deformable cushion as defined in the foregoing and a breast pump device comprising an air passage element, wherein the contacting surface of the pump section of the cushion is in sealing contact to a surface of the air passage element of the breast pump device in a situation of the cushion being positioned with respect to the air passage element of the breast pump device and being either in the initial configuration or the final configuration. The cushion is preferably of such a design that an effective sealing contact of the contacting surface of the pump section of the cushion to the surface of the air passage element is already established as soon as the cushion is positioned with respect to the air passage element while still being in the initial configuration. As suggested earlier, a process of deforming the cushion to the final configuration may involve sliding of the contacting surface of the pump section of the cushion on the surface of the air passage element, so that the sealing contact can be maintained after it has initially been established, wherein the sealing contact is present in all possible configurations of the cushion, which is beneficial to the overall functioning of the breast pump device and contributes to having an optimal effect of an underpressure on both the deforming process as mentioned and the milk expression process.

The invention also relates to a breast pump device, comprising an expression unit including an air passage element. The expression unit of the breast pump device may especially be designed to allow a cushion comprising a valve for at least hindering an airflow through the cushion to be mounted to the air passage element. In such a case, it may be advantageous if the expression unit includes at least one element for receiving and retaining at least one displaceable part of the valve of the cushion in a situation of the valve being opened under the influence of underpressure acting on the cushion from the side of the pump section. The fact is that this allows for having a normally-closed valve in the cushion. The valve can remain closed during a procedure of putting the cushion to the intended mounting arrangement in the air passage element, and can be kept opened after that procedure has taken place in order not to hinder the pressure cycle involved in a pumping session nor a flow of expressed milk in any way.

In a more general sense, the expression unit of the breast pump device may be designed to allow a cushion that is generally sleeve-shaped and that comprises a breast-facing section at one side thereof and a pump section at another side thereof, wherein the pump section includes a contacting surface configured to contact a surface of the air passage element, to be mounted to the air passage element. According to an advantageous option, the expression unit may include an abutment surface for a portion of the pump section to abut against. The portion of the pump section may be an end of the cushion at the side of the pump section, for example. Having an abutment surface in the breast pump device is a practical way of realizing a well-defined mounted position of the cushion on the air passage element and especially determines an end position of the pump section of the cushion in the final configuration of the cushion. In addition to or as an alternative of having an abutment surface, the expression unit of the breast pump device may also have a catch or the like for receiving and securing a portion of the pump section.

It is advantageous if a surface of the air passage element includes a smooth, continuous portion for the contacting surface of the pump section of the cushion to slide on in a situation of the cushion being positioned with respect to the air passage element in an initial configuration and/or position and being deformed and/or moved from the initial configuration and/or position to the final configuration and/or position. In this way, the surface of the air passage element can be adapted to hinder a deforming and/or moving process of the cushion to a minimal extent only. In addition to or as an alternative of the advantageous option of automatically putting a cushion at an appropriate position with respect to an air passage element by allowing the cushion to be deformed, the option of automatically putting a cushion at an appropriate position with respect to an air passage element by allowing at least a part of the cushion to be moved with respect to the air passage element is also covered by the invention.

In a practical embodiment of the breast pump device according to the invention, the breast pump device further comprises i) a vacuum unit configured to realize a pressure cycle in the expression unit, the vacuum unit being connectable to the expression unit and including a pump mechanism configured to suck air from the expression unit, and ii) a controller configured to control operation of the breast pump device during a pumping session, the controller being programmed to drive the pump mechanism for creating a vacuum boost at the start of a pumping session effective to deform and/or move a cushion from an initial configuration and/or position to a final configuration and/or position in a situation of a cushion being positioned with respect to the air passage element. This does not alter the fact that the invention covers the option of driving the pump mechanism in a conventional way, especially when the underpressure that is normally generated for stimulating the breast at the start of a pumping session may be expected to be effective in deforming and/or moving the cushion as an advantageous side effect.

The concept of creating a vacuum boost at the start of a pumping session for the purpose of deforming and/or moving the cushion may be applied independently from the concept of having an abutment surface in the breast pump device, and also from the concept of having at least one element in the breast pump device for receiving and retaining at least one displaceable part of a valve as may be present in a cushion. In case the first concept is applied, indeed, it may be so that the breast pump device comprises a detector that is configured to detect whether or not a cushion is actually present at the air passage element, wherein the controller may be programmed to drive the pump mechanism for creating the vacuum boost only when a cushion is found to be present at the air passage element.

The controller of the breast pump device may be configured to communicate with an external device or system that is separate from the expression unit and the vacuum unit, and that is configured to provide information to a person, including information about a configuration of a cushion and/or a position of a cushion in the air passage element. Practical examples of such a device include a smartphone, a smartwatch, a tablet, a laptop and a computer.

The above-described and other aspects of the invention will be apparent from and elucidated with reference to the following detailed description of four embodiments of a cushion for use with a breast pump device and arrangement at a position between an air passage element of the breast pump device and a user's breast. The description is in the context of extracting human breast milk and a breast pump device adapted to facilitate such a process, which should not be understood so as to imply that the invention is limited to such context.

### BRIEF DESCRIPTION OF THE DRAWINGS

The invention will now be explained in greater detail with reference to the figures, in which equal or similar parts are indicated by the same reference signs, and in which:

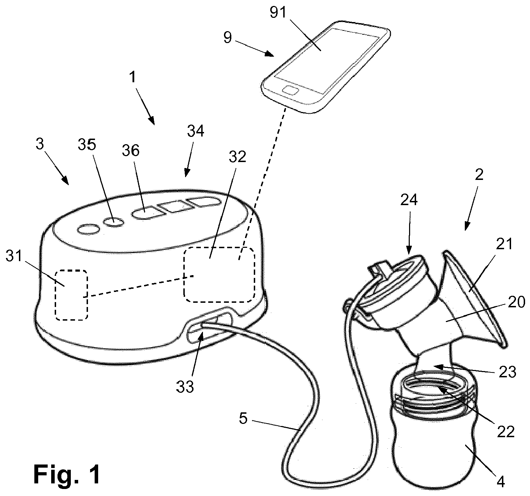

Figure 1 diagrammatically shows a breast pump device comprising an expression kit, a vacuum unit, and a flexible hose interconnecting the expression kit and the vacuum unit, and also diagrammatically shows a smartphone for receiving information from the breast pump device and displaying information to a user;

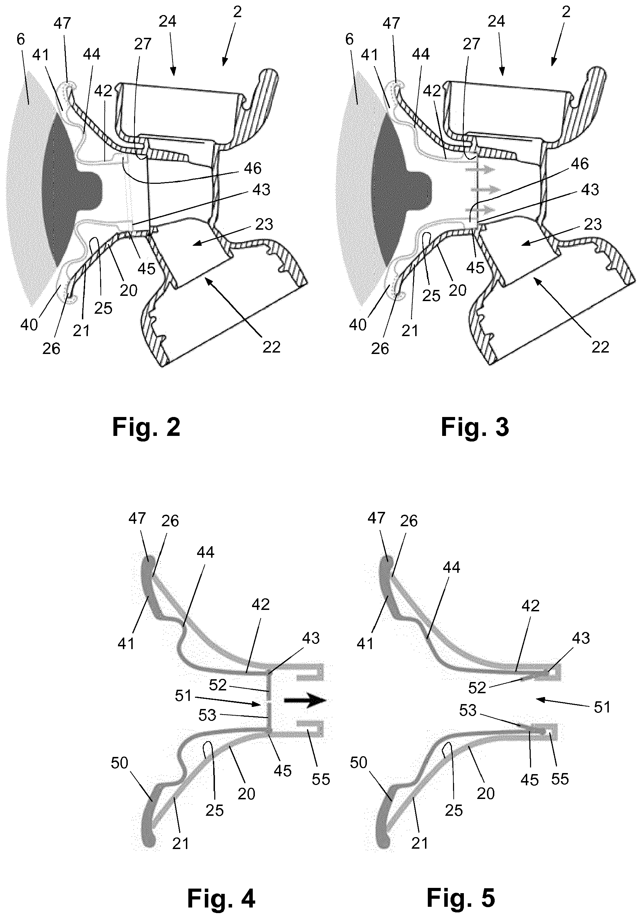

Figures 2 and 3 diagrammatically show a sectional view of an expression kit, a part of a user's breast, and a sectional view of a cushion according to a first embodiment of the invention, as arranged in an air passage element of the expression kit, figure 2 showing the cushion in an initial configuration and figure 3 showing the cushion in a final configuration;
Figures 4 and 5 diagrammatically show a sectional view of a cushion according to a second embodiment of the invention, as arranged in an air passage element, figure 4 showing the cushion in an initial configuration and figure 5 showing the cushion in a final configuration;

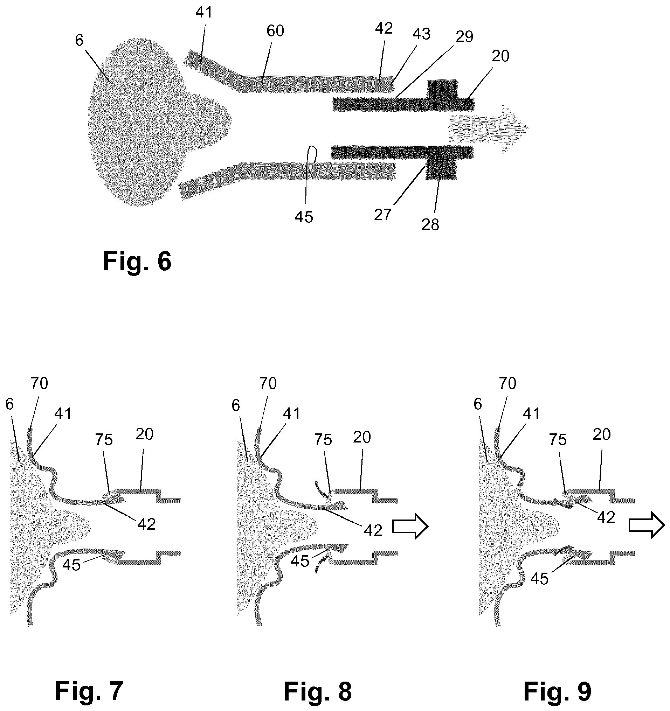

Figure 6 diagrammatically shows a sectional view of an expression kit, a part of a user's breast, and a sectional view of a cushion according to a third embodiment of the invention, as arranged on an air passage element of the expression kit; and
Figures 7-9 diagrammatically show a sectional view of a cushion according to a fourth embodiment of the invention, as arranged in an air passage element, figure 7 showing the cushion in an initial configuration, figure 8 showing the cushion in an intermediate configuration, and figure 9 showing the cushion in a final configuration.

### DETAILED DESCRIPTION OF EMBODIMENTS

The invention is in the field of breast pump devices. With reference to figure 1, a general description of an electric breast pump device will be given first so as to generate a clear picture of contextual aspects of the invention. The invention is also applicable to manually operated breast pump devices.

The breast pump device 1 comprises an expression kit 2 and a vacuum unit 3 for generating a pressure cycle during which vacuum (low pressure) is alternately generated and released. For the purpose of receiving expressed breast milk during operation of the breast pump device 1, a milk receptacle 4 is used which is connectable to the expression kit 2, *e.g.* by screwing, thereby closing a lower end of the expression kit 2. The vacuum unit 3 is an electric vacuum unit and comprises an electric pump 31 and an air valve for realizing an alternating vacuum during operation, i.e. during pumping sessions to be performed by means of the breast pump device 1. The pump 31, the air valve and a controller 32 for realizing proper operation of the pump 31 and the air valve are designed to function in a manner which is well known in the field of breast pump devices. Therefore, further details of these components will not be explained in the present text, and the same is applicable to other practical aspects of the vacuum unit 3 known per se. The pump 31 is electrically connected to a source of electric power, which may be the mains or a battery, for example. The pump 31 and the controller 32 are diagrammatically depicted in figure 1 as dashed rectangles, whilst the air valve is not shown. A path allowing for exchange of control signals etc. between the pump 31 and the controller 32 is diagrammatically depicted in figure 1 as a dashed line. The path may be realized as any suitable type of communication wire, for example.

The expression kit 2 comprises an air passage element 20 including a breast-receiving funnel 21, and further comprises an aperture acting as a milk outlet 22, and a milk path 23 from the breast-receiving funnel 21 to the milk outlet 22. The breast-receiving funnel 21 is thus in fluid communication with the milk outlet 22 through the milk path 23.

In figure 1, the breast pump device 1 is shown in an assembled condition, in which the vacuum unit 3 is connected to the expression kit 2 through a flexible hose 5, wherein an air inlet 33 of the vacuum unit 3 is connected to an air outlet 24 of the expression kit 2. Such a configuration allows for a remote arrangement of the vacuum unit 3 with respect to the expression kit 2, so that the size of the part of the breast pump device 1 which is to be applied to a woman's breast can be kept within reasonable limits. It is to be noted that the breast pump device 1 can comprise two expression kits 2 for enabling a lactating woman using the breast pump device 1 to extract milk from two breasts at the same time, in which case the expression kits 2 can share a common vacuum unit 3.

In general, the breast pump device 1 is used to realize milk expression from a woman's breast. To that end, an alternating vacuum (pressure cycle) is applied to the breast. During periods of low pressure, or vacuum, the actual process in which milk is expressed from the breast takes place. Every time that the vacuum is released, freshly expressed milk drops in the receptacle 4.

Advantageously, as shown, the breast pump device 1 comprises a user interface 34 for allowing a user to control operation of the breast pump device 1. In the shown example, the user interface 34 is arranged on the vacuum unit 3 and enables a user to provide input to the controller 32. The user interface 34 may be realized in any suitable manner such as through a number of buttons as shown, or through a touch screen, for example. By way of example, it is noted that the user interface 34 may comprise one button 35 for activating a stimulation mode and three buttons 36 for choosing one of three expression settings.

Besides the breast pump device 1, figure 1 shows a smartphone 9 that may be used for providing information during operation of the breast pump device 1 to a user. In particular, the smartphone 9 may be used to run an application designed to display relevant information about the breast pump device 1 and pumping sessions performed by means of the breast pump device 1 on the screen 91 of the smartphone 9. Also, the smartphone 9 may be suitable to receive input from a user and transmit the input to the controller 32, in addition to or as an alternative of the user interface 34 on the vacuum unit 3. A path allowing for exchange of information between the controller 32 and the smartphone 9 is diagrammatically depicted in figure 1 as a dashed line. The path is preferably realized in a wireless manner, which should not be understood such as to imply that a wired path is not covered by the invention as well.

In order to provide a soft and warm feel to the breast, it is advantageous to avoid direct contact between the breast and the air passage element 20. To that end, a cushion is provided, which is intended to be mounted to the air passage element 20 by a user prior to operating the breast pump device 1 for performing a pumping session. Figures 2 and 3 relate to a first embodiment of a cushion as mentioned, figures 4 and 5 relate to a second embodiment of such a cushion, and figure 6 relates to a third embodiment of such a cushion.

In both figures 2 and 3, an expression kit 2, a part of a user's breast 6, and a sectional view of the cushion 40 according to the first embodiment of the invention as arranged in the air passage element 20 of the expression kit 2 are shown. The cushion 40 is generally sleeve-shaped. In the shown example, the air passage element 20 includes a breast-receiving funnel 21 having a substantially circular periphery, and the cushion 40 has a substantially circular periphery as well. The cushion 40 comprises a breast-facing section 41 at one side thereof and a pump section 42 at another side thereof.

The cushion 40 is intended to be arranged in the air passage element 20 by a user in the configuration as shown in figure 2, which is referred to as initial configuration. When the breast pump device 1 of which the expression kit 2 is part is operated and an underpressure is generated at a back side of the air passage element 20, as diagrammatically indicated by means of arrows in figure 3, another configuration of the cushion 40 is realized, which is referred to as final configuration. In this way, it is achieved that the user is not required to perform complex handling of the cushion 40 aimed at putting the cushion 40 in the intended mounting arrangement in the air passage element 20 as shown in figure 3, in which an end 43 of the cushion 40 at the side of the pump section 42 is rather deep down in the air passage element 20, as this is done automatically under the influence of the underpressure once the user has inserted the cushion 40 in the air passage element 20 from the front side to a limited depth only. In order to allow for this advantageous effect, the cushion 40 and the expression kit 2 have features as will be explained in the following.

In the first place, it is to be noted that the cushion 40 according to the first embodiment of the invention is stretchable, in a generally axial direction. The cushion 40 comprises a flexible material such as silicone and includes a stretching section 44 at a position between the breast-facing section 41 and the pump section 42. As can be derived from a comparison between figures 2 and 3, the stretching section 44 is deformable between a more compact configuration and a more straightened configuration. In the shown example, the stretching section 44 comprises a curved portion that is flattened when the cushion 40 is stretched from the initial configuration to the final configuration. Especially at the position of the cushion 40, the flexibility of the cushion 40 may be high, which contributes to enabling the stretching process under the influence of underpressure generated by the vacuum unit 3 of the breast pump device 1 of which the expression kit 2 is part.

The pump section 42 of the cushion 40 includes a contacting surface 45 configured to contact an interior surface 25 of the air passage element 20. In the shown example, the pump section 42 has a stiffened annular portion 46 in the form of a thickened end portion, and the contacting surface 45 is formed by an exterior surface of the stiffened annular portion 46. When the cushion 40 has been placed in the air passage element 20 by the user, an annular sealing contact between the pump section 42 and the air passage element 20 is realized at the position of the contacting surface 45 of the pump section 42. This sealing contact is maintained when the cushion 40 is put from the initial configuration to the final configuration, wherein the contacting surface 45 slides on the interior surface 25 of the air passage element 20 in the stretching process of the cushion 40. On the basis of the sealing contact, especially the fact that the sealing contact may be present from the start, the extent to which underpressure may be effective in stretching the cushion 40 may be optimal. In order to facilitate the sliding process of the contacting surface 45 of the pump section 42 on the interior surface 25 of the air passage element 20, it is advantageous for the interior surface 25 of the air passage element 20 to be smooth and continuous, i.e. free from unevenness in the form of steps, sharp curves etc.

At the front side, the position of the cushion 40 with respect to the air passage element 20 is fixable as the breast-facing section 41 includes a coupling portion 47 comprising a groove for accommodating a peripheral front edge 26 of the air passage element 20. At the back side, a position of the end 43 of the cushion 40 at the side of the pump section 42 in the final configuration of the cushion 40 with respect to the air passage element 20 is determined by an abutment surface 27 located in the expression kit 2. In the shown example, the abutment surface 27 is present at the position of a step in the interior surface 25 of the air passage element 20, and has an annular appearance.

As already explained in the foregoing, the functioning of the cushion 40 according to the first embodiment relies on the cushion 40 being stretchable. The cushion 40 is initially put in place in the air passage element 20 in a compact, initial configuration, wherein the cushion 40 is coupled to the peripheral front edge 26 of the air passage element 20. However, a user is not required to ensure that the cushion 40 is stretched down the air passage element 20 in order to realize an intended mounting arrangement of the cushion 40 in the air passage element 20, as this is done automatically under the influence of underpressure. In view of the fact that a breast pump device 1 is normally equipped to create underpressure in order to realize the pressure cycle required during a pumping session, no additional components are needed in the design of the breast pump device 1, which is a notable advantage of the invention. The cushion 40 is designed so as to ensure sealing contact between the contacting surface 45 of the pump section 42 and the interior surface 25 of the air passage element 20 already in the initial configuration of the cushion 40. During the stretching process, the contacting surface 45 of the pump section 42 slides on the interior surface 25 of the air passage element 20 until the end 43 of the cushion 40 at the side of the pump section 42 abuts against the abutment surface 27 of the air passage element 20. Thus, the invention provides a most convenient way of putting a cushion 40 to a predetermined, correct mounting position, i.e. a mounting position as foreseen, on the air passage element 20 without requiring specific effort from a user.

Figures 4 and 5 relate to a second embodiment of a cushion 50 according to the invention. The cushion 50 according to the second embodiment is actually an alternative of the cushion 40 according to the first embodiment, a notable difference between the two cushions 40, 50 residing in the fact that the cushion 50 according to the second embodiment is equipped with a valve 51, whereas the cushion 40 according to the first embodiment is not. In the shown example, the valve 51 is arranged at the end 43 of the cushion 50 at the side of the pump section 42, and is of the normally-closed type. In this way, it can be achieved that in the initial configuration of the cushion 50, as shown in figure 4, the underpressure that is exerted in order to put the cushion 50 to the final configuration, as diagrammatically indicated by means of an arrow in the figure, can be used to that effect in an optimal manner, as leakage of air through the cushion 50 is prevented. In the process, it is not necessary for the cushion 50 to be closed by means of a breast 6 at the side of the breast-facing section 41.

When a valve 51 as described in the foregoing is applied, it is advantageous if the breast pump device 1 is designed so as to ensure that an operational, open appearance of the cushion 50 is maintained once the cushion 50 has been sucked to place in the air passage element 20 and does not need to be stretched any further. In view thereof, the air passage element 20 may be equipped with at least one suitable element 55 for receiving and retaining at least one displaceable part of the valve 51 in a situation of the valve 51 being opened under the influence of an underpressure prevailing at the side of the pump section 42 that is effective to that end. In the shown example, the element 55 comprises an annular internal channel in the air passage element 20, which is arranged to receive and retain flexible flaps 52, 53 of the valve 51.

It follows from the foregoing that it is very well possible to use a valve 51 for ensuring an effective process of putting the cushion 50 from the initial configuration to the final configuration in a first instance, as there is no need for taking complex measures for avoiding a hindering effect of such a valve on an airflow and a flow of expressed milk during a subsequent pumping session.

Figure 6 relates to a third embodiment of a cushion 60 according to the invention. In order to distinguish the cushion 60 of the third embodiment from the cushion 40 of the first embodiment and the cushion 50 of the second embodiment, the cushion 60 of the third embodiment is referred to as outside cushion 60.

The outside cushion 60 is designed to be arranged on the air passage element 20 at the outside thereof. A user only needs to place the outside cushion 60 over the air passage element 20 to a limited extent. It will be understood that the outside cushion 60 is especially suitable to be used with air passage elements 20 having a more or less straight outside appearance, at least at a front side thereof, which does not alter the fact that the outside cushion 60 can be used with air passage elements 20 which are of a different design as well.

The underpressure as can be generated in the air passage element 20 for the purpose of sucking air from the air passage element 20 in a direction away from the breast 6 is diagrammatically indicated by means of an arrow in figure 6. When the outside cushion 60 is in the initial position as shown in figure 6 and the underpressure is applied, the outside cushion 60 is pulled further down the air passage element 20 until the end 43 of the cushion 60 at the side of the pump section 42 abuts against an abutment surface 27 of the air passage element 20, which is provided as the surface of an outside annular ridge 28 of the air passage element 20 in the shown example. In the case of the outside cushion 60, the contacting surface 45 of the pump section 42 is constituted by the portion of the interior surface of the pump section 42 contacting an exterior surface 29 of the air passage element 20 and sliding on the exterior surface 29 of the air passage element 20 during the process of the outside cushion 60 assuming the final configuration on the air passage element 20.

In the case of the outside cushion 60, the process of putting the outside cushion 60 to a predetermined, correct mounting arrangement on the air passage element 20 from a first mounting arrangement realized by a user may involve one of stretching of the outside cushion 60 and moving the outside cushion 60 in its entirety, or a combination of these two effects as may be invoked under the influence of underpressure, depending on various factors including the stiffness characteristics of the outside cushion 60.

Figures 7-9 relate to a fourth embodiment of a cushion 70 according to the invention. In the following, the cushion 70 of the fourth embodiment is referred to as radially flexing cushion 70. The radially flexing cushion 70 is intended to be used with an air passage element 20 having a flexible flip portion 75 at the front side thereof. In the shown example, the flexible flip portion 75 is of annular shape.

In the initial configuration of the radially flexing cushion 70, the flexible flip portion 75 is in an initial, default condition, and encompasses an end portion of the pump section 42 of the cushion 70 while being contacted by the contacting surface 45 of the pump section 42, as shown in figure 7. When underpressure is exerted from the side of the pump section 42, as diagrammatically indicated by means of an arrow in both figures 8 and 9, the radially flexing cushion 70 is made to collapse to some extent in the radial direction and is also pulled in a backward direction to some extent, as a result of which the flexible flip portion 75 of the air passage element 20 is made to perform an inwardly directed flexing movement. Further from that position, as the backward moving/stretching of the cushion 70 is continued, the flexible flip portion 75 flips to a folded configuration as illustrated in figure 9.

The flipping action of the flexible flip portion 75 is diagrammatically indicated by means of arrows in figures 8 and 9. Once the flexible flip portion 75 is in the folded configuration, the radially flexing cushion 70 is tightly fitted in the air passage element 20, in a mounting arrangement as intended. As may be derived from figures 7-9, it may be beneficial to the process of causing the flexible flip portion 75 to flip if a surface of the radially flexing cushion 70 facing the flexible flip portion 75 has an indent or is otherwise appropriately shaped for allowing the cushion 70 to take along an edge of the flexible flip portion 75 while flexing/moving under the influence of underpressure.

In general, in respect of the cushion 70 according to the fourth embodiment of the invention, it is noted that the breast pump device 1 may be equipped with a flexible portion that is arranged and configured to contact the cushion 70 and to be deformed under the influence of underpressure and/or deformation/movement of the cushion 70.

The smartphone 9 as shown in figure 1 or another suitable device can be used to provide information about a cushion to a user. For example, in case an abutment surface 27 for a portion of the pump section 42 to abut against is present in an air passage element 20, a sensor (not shown) may be associated with the abutment surface 27 and the user may be provided with a confirmation that the cushion has been sucked to the correct mounting arrangement on the air passage element 20 as soon as it is sensed that the abutment surface 27 is touched by the cushion.

It will be clear to a person skilled in the art that the scope of the invention is not limited to the examples discussed in the foregoing, but that several amendments and modifications thereof are possible without deviating from the scope of the invention as defined in the attached claims. It is intended that the invention be construed as including all such amendments and modifications insofar they come within the scope of the claims or the equivalents thereof. While the invention has been illustrated and described in detail in the figures and the description, such illustration and description are to be considered illustrative or exemplary only, and not restrictive. The invention is not limited to the disclosed embodiments. The drawings are schematic, wherein details which are not required for understanding the invention may have been omitted, and not necessarily to scale.

Variations to the disclosed embodiments can be understood and effected by a person skilled in the art in practicing the claimed invention, from a study of the figures, the description and the attached claims. In the claims, the word "comprising" does not exclude other steps or elements, and the indefinite article "a" or "an" does not exclude a plurality. Any reference signs in the claims should not be construed as limiting the scope of the invention.

Elements and aspects discussed for or in relation with a particular embodiment may be suitably combined with elements and aspects of other embodiments, unless explicitly stated otherwise. Thus, the mere fact that certain measures are recited in mutually different dependent claims does not indicate that a combination of these measures cannot be used to advantage.

The term "comprise" as used in this text will be understood by a person skilled in the art as covering the term "consist of'. Hence, the term "comprise" may in respect of an embodiment mean "consist of', but may in another embodiment mean "contain/include at least the defined species and optionally one or more other species".

Notable aspects of the invention can be summarized as follows. Complexity of a process of mounting a cushion 40, 50, 60, 70 to an air passage element 20 of a breast pump device 1 is decreased by only requiring a user to arrange the cushion 40, 50, 60, 70 on the air passage element 20 in a first arrangement in which the cushion 40, 50, 60, 70 is easily accessible. Particularly, the user does not need to perform cumbersome actions aimed at accurately putting the cushion 40, 50, 60, 70 to an intended, correct position, as a predetermined mounting arrangement of the cushion 40, 50, 60, 70 on the air passage element 20 is realized under the influence of underpressure prevailing at a back side of the air passage element 20. In the process, the cushion 40, 50, 60, 70 is put to the predetermined mounting arrangement, wherein the cushion 40, 50, 60, 70 is made to flex and/or move.

## Claims

1. Cushion (40, 50, 60, 70) configured to be mounted to an air passage element (20) of a breast pump device (1) and to contact a breast (6) of a user of the breast pump device (1),
- the cushion (40, 50, 60, 70) generally being sleeve-shaped,
- the cushion (40, 50, 60, 70) comprising a breast-facing section (41) at one side thereof and a pump section (42) at another side thereof, wherein the pump section (42) includes a contacting surface (45) configured to contact a surface (25, 29) of the air passage element (20), and
- the cushion (40, 50, 60, 70) further being configured to be deformable from an initial configuration to a final configuration when the cushion (40, 50, 60, 70) is brought under the influence of underpressure acting on the cushion (40, 50, 60, 70) from the side of the pump section (42).

2. Cushion (40, 50, 60, 70) according to claim 1, being configured to be at least partially arranged in an air passage element (20) of a breast pump device (1) and/or to be arranged to at least partially encompass an air passage element (20) of a breast pump device (1), wherein the contacting surface (45) is configured to contact an interior surface (25) of the air passage element (20) and/or to contact an exterior surface (29) of the air passage element (20).

3. Cushion (40, 50, 60) according to claim 1 or 2, being configured to be stretchable in a generally axial direction under the influence of underpressure acting on the cushion (50) from the side of the pump section (42).

4. Cushion (40, 50) according to claim 3, including a stretching section (44) at a position between the breast-facing section (41) and the pump section (42), the stretching section (44) being deformable between a more compact configuration and a more straightened configuration and thereby being configured to facilitate stretching of the cushion (40).

5. Cushion (40, 50) according to any of claims 1-4, wherein the pump section (42) includes a stiffened annular portion (46), the contacting surface (45) being constituted by at least a portion of a surface of the pump section (42) at a position of the stiffened annular portion (46).

6. Cushion (40, 50) according to any of claims 1-5, wherein the breast-facing section (41) includes a coupling portion (47) configured to engage with the air passage element (20).

7. Cushion (40, 50) according to claim 6, wherein the coupling portion (47) comprises a groove configured to receive and accommodate a peripheral front edge (26) of the air passage element (20).

8. Cushion (50) according to any of claims 1-7, comprising a valve (51) located in the cushion to at least hinder an airflow through the cushion.

9. Assembly of a cushion (40, 50, 60, 70) according to any of claims 1-8 and a breast pump device (1) comprising an air passage element (20), wherein the contacting surface (45) of the pump section (42) of the cushion (40, 50, 60, 70) is in sealing contact to a surface (25, 29) of the air passage element (20) of the breast pump device (1) in a situation of the cushion (40, 50, 60, 70) being positioned with respect to the air passage element (20) of the breast pump device (1) and being either in the initial configuration or the final configuration.

10. Breast pump device (1), comprising an expression unit (2) including an air passage element (20), the expression unit (2) being designed to allow a cushion (50) according to claim 8 to be mounted to the air passage element (20), and the expression unit (2) including at least one element (55) for receiving and retaining at least one displaceable part (52, 53) of the valve (51) of the cushion (50) in a situation of the valve (51) being opened under the influence of underpressure acting on the cushion (50) from the side of the pump section (42).

11. Breast pump device (1), comprising an expression unit (2) including an air passage element (20), the expression unit (2) being designed to allow a cushion (40, 60) that is generally sleeve-shaped and that comprises a breast-facing section (41) at one side thereof and a pump section (42) at another side thereof, wherein the pump section (42) includes a contacting surface (45) configured to contact a surface (25, 29) of the air passage element (20), to be mounted to the air passage element (20), and the expression unit (2) including an abutment surface (26) for an portion of the pump section (42) to abut against.

12. Breast pump device (1) according to claim 10 or 11, wherein a surface (25, 29) of the air passage element (20) includes a smooth, continuous portion for the contacting surface (45) of the pump section (42) of the cushion (40, 50, 60) to slide on in a situation of the cushion (40, 50, 60) being positioned with respect to the air passage element (20) in an initial configuration and/or position and being deformed and/or moved from the initial configuration and/or position to a final configuration and/or position.

13. Breast pump device (1) according to any of claims 10-12, further comprising
- a vacuum unit (3) configured to realize a pressure cycle in the expression unit (2), the vacuum unit (3) being connectable to the expression unit (2) and including a pump mechanism (31) configured to suck air from the expression unit (2), and
- a controller (32) configured to control operation of the breast pump device (1) during a pumping session, the controller (32) being programmed to drive the pump mechanism (31) for creating a vacuum boost at the start of a pumping session effective to deform and/or move a cushion (40, 50, 60) from an initial configuration and/or position to a final configuration and/or position in a situation of a cushion (40, 50, 60) being positioned with respect to the air passage element (20).

14. Breast pump device (1), comprising
- an expression unit (2) including an air passage element (20), the expression unit (2) being designed to allow a cushion (40, 50, 60, 70) that is generally sleeve-shaped and that comprises a breast-facing section (41) at one side thereof and a pump section (42) at another side thereof, wherein the pump section (42) includes a contacting surface (45) configured to contact a surface (25, 29) of the air passage element (20), to be mounted to the air passage element (20),
- a vacuum unit (3) configured to realize a pressure cycle in the expression unit (2), the vacuum unit (3) being connectable to the expression unit (2) and including a pump mechanism (31) configured to suck air from the expression unit (2), and
- a controller (32) configured to control operation of the breast pump device (1) during a pumping session, the controller (32) being programmed to drive the pump mechanism (31) for creating a vacuum boost at the start of a pumping session effective to deform and/or move a cushion (40, 50, 60, 70) from an initial configuration and/or position to a final configuration and/or position in a situation of a cushion (40, 50, 60, 70) being positioned with respect to the air passage element (20).

15. Breast pump device (1) according to claim 13 or 14, wherein the controller (32) is configured to communicate with an external device or system (9) that is separate from the expression unit (2) and the vacuum unit (3), and that is configured to provide information to a person, including information about a configuration of a cushion (40, 50, 60, 70) and/or a position of a cushion (40, 50, 60, 70) in the air passage element (20).
